# EUROPÄISCHE PATENTANMELDUNG

(11) **EP 0 826 962 A1**
(43) Veröffentlichungstag der Anmeldung: **04.03.1998**
(21) Anmeldenummer: 97114034.8
(22) Anmeldetag: 14.08.1997
(51) Int. Cl.: G01N 27/327, C12Q 1/00, C12Q 1/28

(54) **Dickschichtsensor zur Bestimmung der Peroxidaseaktivität**

(30) Priorität: 14.08.1996 DE 19632778
(71) Anmelder: Gesellschaft für Biotechnologische Forschung mbH (GBF), D-38124 Braunschweig (DE)
(72) Erfinder: Stiene, Matthias, 38124 Braunschweig (DE); Bilitewski, Ursula, Dr., 38124 Braunschweig (DE)
(74) Vertreter: Boeters, Hans Dietrich, Dr.

(57) **Zusammenfassung**

Bekannte Verfahren zum Nachweis hochmolekularer Substanzen mit Proteinen, Mikroorganismen, Hormonen, aber auch von niedermolekularen Verbindungen wie Pestiziden und anderen Schadstoffen haben den Nachteil, daß sie entweder komplex, oder nicht einfach und kostengünstig als Massenprodukt zum Einsatz als Einmal-Elektrode hergestellt werden können.

In dem erfindungsgemäßen Dickschichtsensor zur Bestimmung der Peroxidase-Aktivität werden zur Herstellung der sensitiven Elektrode Widerstandspasten verwendet, die dadurch bestimmt werden, daß sie geringe Mengen p-Benzochinon in der Gegenwart von hohen Konzentrationen von H₂0₂ messen können. Als Pasten werden spezielle, siebdruckfähige Graphit- bzw. Metalloxidpasten verwendet.

## Beschreibung

Die Erfindung betrifft einen Dickschichtsensor zur elektrochemischen Detektion von Peroxidase, sowie ein Verfahren zu ihrer Herstellung.

Immunoassays werden zum Nachweis von hochmolekularen Substanzen, wie Proteinen, Mikroorganismen, Hormonen, aber auch von niedermolekularen Verbindung wie Pestiziden und anderen Schadstoffen eingesetzt. Gängiges Standard eines derartigen Nachweises ist der ELISA, bei dem enzymmarkierte Antikörper oder Antigene verwendet werden. Die Wirkungsweise des ELISA (Enzyme-Link Immuno Sorbent Assay) ist beispielsweise in P. Tijssen: "Practice and Theorie of Enzyme Immunoassays", Elsevier - Amsterdam, New York, Oxford, 1985 beschrieben. Der Nachweis des Analyten erfolgt letztendlich über die Bestimmung der gebundenen Enzymmenge. Häufig verwendete Enzyme sind die Peroxidase, alkalische Phosphatase und β-Galactosidase. Es werden zum Nachweis des Enzyms in der Regel Substrate verwendet, die zu einer Änderung der optischen Dichte bzw. Fluoreszenz führen, d.h. die zur Detektion eingesetzten Meßgeräte sind in der Regel Photometer oder Fluorimeter.

Verglichen mit dem geschilderten Nachweisverfahren sind elektrochemische Methoden in der Regel preisgünstiger bezüglich der Anschaffung und im Dauerbetrieb, da beispielsweise kein Wechsel von Lampen anfällt. Allerdings fehlen bislang die entsprechenden Immunoassays, was mehrere Gründe hat:
- Viele der verwendeten "elektrochemischen" Enzyme besitzen, wie im Fall der Oxidasen, eine geringere Aktivität als die "klassischen" Enzymmarker.
- Die elektrochemisch aktiven Substrate für die alkalische Phosphatase sind kommerziell nicht erhältlich und wenig stabil.
- Für die Peroxidase gibt es bisher keine geeignete Kombination von Substrat und Elektrode, welche auch als "Einmal"-Sensor verwendet werden könnte.

In "Aizawa et al.: Enzyme Immuno Sensor, II. Electrochemical Determination of IgG with an Antibody Bound-Membrane", Journal of Membrane Science, 4, 1979, 221 - 228 und Boitieux et al.: "An Antibody Electrode, Preliminary Report on a New Approach in Enzyme Immunoassay", Clin. Chem. 25/2, Seite 318 - 321 (1979) wurden erste Versuche publiziert, einen ELISA auf die Oberfläche einer Elektrode zu übertragen. Erreicht werden soll dabei die Integration von Detektor und Immunreaktor auf engstem Raum, so daß diese Tests, d.h. der Nachweis von hochmolekularen Substanzen, nicht mehr an stationäre Geräte wie ELISA-Reader und Washer gebunden sind. Weiterhin wird eine Minimierung der Meßdauer auf die Größenordnung von mehreren Minuten angestrebt, was im Vergleich zu den Standard-ELISA-Prozeduren, für die mehrere Stunden benötigt werden, einen erheblichen Zeitgewinn darstellt, solange die Möglichkeit der parallelen Detektion von 96 Proben auf einer ELISA-Platte nicht berücksichtigt wird. In diesem Sinne empfehlen sich die angestrebten Elektroden für örtlich flexible Messungen bei geringem apparativen Aufwand und kleinem Probenaufkommen und nicht für größere Screening-Projekte. Eine gemeinsame Eigenschaft dieser Elektroden-Prototypen ist jedoch, daß sie nicht in einem kostengünstigen und für die Massenproduktion geeigneten Prozeß hergestellt werden können. Hierdurch schließt sich sowohl die routinemäßige Anwendung dieser Technik als auch die Verwendung der Elektroden als "Einmal-Sensoren" aus.

Weiterhin ist bekannt, Elektroden mit Hilfe der Dickschichttechnik kostengünstig und in großen Serien zu produzieren, wie es für Enzymelektroden, beispielsweise zur Blutzuckerbestimmung, bekannt ist, und die sich seit geraumer Zeit im Handel befinden. Einen Überblick über die Massenproduktion derartiger Biosensoren ist in Bilitewski et al. "Mass Production of Biosensors" Analytical Chemistry (1993, 65, 525A - 533A beschrieben).

Aufgrund der vielen kommerziell erhältlichen Antikörper-Konjugate, der hohen Aktivität und Stabilität ist die Peroxidase für die Entwicklung von Immunoassays eines der interessantesten Markerenzyme. Die Detektion der Peroxidaseaktivität erfolgt dabei im allgemeinen über die Zersetzung von Wasserstoffperoxid und einem Cosubstrat, welches für die Änderung der optischen Dichte oder der Fluoreszenz verantwortlich ist. Bei geeigneter Kombination von Elektrode und Cosubstrat (Mediator) kann man die vielen Vorteile der Peroxidase, die sie zu dem weitverbreitetsten Enzymmarker für die optische Detektion werden ließ, auch für die elektrochemische Detektion nutzen. Ein derartiges Verfahren ist in Skládal et al.: "A disposable amperometric immunosensor for 2.4-dichlorophenoxyacetic acid, Analytica Chimica Acta 304 (1995), Seite 361 - 368 beschrieben, wobei die Peroxidase als Enzymmarker und ein Dickschichtsensor als Detektor benutzt wurde. Dabei wurde Platin oder Gold als Material für die Elektrode benutzt, das, wie in der Dickschichttechnik üblich, im Siebdruck aufgebracht und anschließend gebrannt wurde. Allerdings weisen diese Edelmetallelektroden für die Bestimmung von der Peroxidaseaktivität nicht die idealen elektrochemischen Eigenschaften auf, da sie aufgrund der Anwesenheit von H₂O₂ einen hohen Grundstrom zeigen. Daher erfordert die selektive Bestimmung der Peroxidaseaktivität eine Elektrode mit speziellen Oberflächeneigenschaften. Es hat sich nun herausgestellt, daß bei dem konventionellen Elektrodenmaterial nur sehr glattes, hydrophobes glassy Carbon als geeignet angesehen werden kann. Allerdings ist glassy Carbon nicht siebdruckfähig, so daß eine preisgünstige Herstellung von Einmalelektroden nicht gegeben ist.

Der Erfindung liegt daher die Aufgabe zugrunde, einen Dickschichtsensor zur Bestimmung der Peroxidaseaktivität zu schaffen, bei dem das Verhältnis zwischen enzymatischem und nichtenzymatischem Signal günstig ist und der als Einmal-Sensor verwendet werden kann.

Die Aufgabe wird durch die Merkmale des unabhängigen Anspruchs 1 gelöst. Bevorzugte Ausgestaltungen der Erfindung sind Gegenstand der Unteranspüche.

Die vorliegende Erfindung betrifft einen Dickschichtsensor zur Bestimmung der Peroxidaseaktivität mit einer sensitiven Elektrode, wobei die Elektrode aus einer Widerstandspaste hergestellt und die Widerstandspaste dadurch bestimmt wird, daß sie geringe Menge p-Benzochinon in der Gegenwart von hohen Konzentrationen von H₂O₂ messen kann. Ein zusätzlicher Selektionsparameter ist dadurch gegeben, daß die Widerstandspaste geringe Mengen p-Benzochinon in der Gegenwart hoher Konzentrationen von Hydrochinon messen kann.

Vorzugsweise beträgt das Elektrodenpotential der erfindungsgemäßen Dickschichtsensor zwischen -10 und -700 mV in Abhängigkeit von dem vrwendeten Pastentyp und der Referenzelektrode.

Vorzugsweise ist die Widerstandspaste eine graphithaltige Paste und weist einen relativ niedrigen eigenen Widerstand auf. Es kann auch eine Metalloxidpaste verwendet werden, die nicht H₂O₂-empfindlich ist. Dabei wird vorzugsweise eine Blei-Rutenium-Dioxidpaste gewählt.

Vorzugsweise sind die Widerstandspasten siebdruckfähig, so daß preisgünstige Elektroden und damit Sensoren herstellbar sind, die als "Einmal"-Sensoren verwendet werden können.

Ferner umfaßt die vorliegende Erfindung ein Verfahren zur Herstellung eines Dickschichtsensors zur Bestimmung der Peroxidaseaktivität mit sensitiven Elektroden, wobei nach den obigen Kriterien Widerstandspasten verwendet werden, diese mittels Siebdruck auf einen Träger aufgebracht und anschließend getrocknet bzw. gebrannt werden. Bei der Verwendung geeigneter Graphitpasten wird üblicherweise eine Trocknungstemperatur von mindestens 80° C verwendet. Vorzugsweise beträgt die Trocknungstemperatur (Einbrenntemperatur) 150° C. Bei einer Metalloxidpaste wird eine Brenntemperatur von 700°C bis 900°C und mehr verwendet, entsprechend der Vorschrift der jeweiligen Paste. Vorzugsweise wird eine Brenntemperatur von ca. 850°C verwendet.

Vorteilhafterweise kann daher der erfindungsgemäße Dickschichtsensor (auch als Hybridtransducer bezeichnet) zur Bestimmung der Peroxidaseaktivität im Siebdruckverfahren hergestellt werden und kann daher zu einem Stückpreis produziert werden, bei dem auch der Einsatz als "Einmal-Sensor" möglich ist. Die für die Herstellung der sensitiven Elektrodenoberfläche verwendeten Materialien sind speziell ausgewählte Widerstands-, Graphit- oder Carbonpasten, die normalerweise zur Produktion von Dickschichtwiderständen in elektronischen Schaltungen verwendet werden, so daß diese ebenfalls preisgünstig zu erhalten sind.

Bevorzugte Ausführungsformen der Erfindung werden anhand der beigefügten Zeichnungen beschrieben, in denen:
Fig. 1 den schematischen Ablauf der enzymatischen Reaktion und der anschließenden elektrochemischen Detektion des p-Benzochinons beschreibt,
Fig. 2a und Fig. 2b den schematischen Aufbau eines erfindungsgemäßen Dickschichtsensors in perspektivischer Ansicht und im Querschnitt zeigt,
Fig. 3 ein zyklisches Voltamogramm eines Dickschichtsensors unter Verwendung der Carbonpaste Ercon C-458 zeigt,
Fig. 4 den Signalanstieg nach Zugabe gelöster Peroxidase im Falle der Carbonpaste Ercon C-458 zeigt,
Fig. 5 ein zyklisches Voltamogramm eines Dickschichtsensors unter Verwendung der Metalloxidpaste Du Pont QS872 zeigt,
Fig. 6 den Signalanstieg nach Zugang gelöster Peroxidase im Falle der Metalloxidpaste Du Pont QS872 zeigt,
Fig. 7 ein zyklisches Voltamogramm eines Dickschichtsensors unter Verwendung der Carbonpaste Acheson Electrodag 421 SS zeigt,
Fig. 8 die Verschiebung der zyklischen Voltamogramme eines Dickschichtsensors nach Fig. 7 bei der Verwendung unterschiedlicher Referenzelektroden zeigt,
Fig. 9 ein zyklisches Voltamogramm eines Dickschichtsensors unter Verwendung der Carbonpaste Ercon G-448 mit einer Goldschicht zeigt,
Fig. 10 eine Gegenüberstellung der zyklischen Voltamogramme des Dickschichtsensors nach Fig. 9 einmal mit und einmal ohne Goldschicht zeigt,
Fig. 11 ein zyklisches Voltamogramm eines Dickschichtsensors unter Verwendung der Carbonpaste Ercon G-450 mit einer Goldschicht zeigt,
Fig. 12 eine Gegenüberstellung der zyklischen Voltamogramme des Dickschichtsensors nach Fig. 11 einmal mit und einmal ohne Goldschicht zeigt,
Fig. 13 ein zyklisches Voltamogramm eines Dickschichtsensors unter Verwendung der Carbonpaste Ercon Exp #551112 mit einer Goldschicht zeigt, und
Fig. 14 eine Gegenüberstellung der zyklischen Voltamogramme des Dickschichtsensors nach Fig. 13 einmal mit und einmal ohne Goldschicht zeigt.

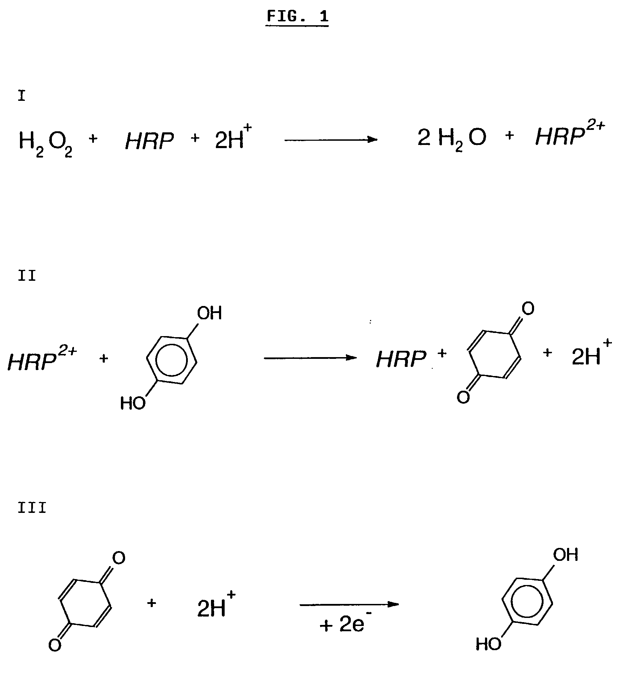

Fig. 1 zeigt den schematisierten Ablauf der enzymatischen Reaktion und der anschließenden elektrochemischen Detektion des p-Benzochinons. Wie bereits oben ausgeführt, erfolgt die Bestimmung der Antikörper- bzw. Antigenkonzentration über die Detektion von Enzymmengen, wobei der Mechanismus einer elektrochemischen Detektion wie folgt abläuft: das Enzym Peroxidase, hier mit HRP (horse radish peroxide) bezeichnet, katalysiert die Zersetzung von H₂O₂ in Gegenwart von einem Hydriddonor ( hier Hydrochinon, Teilschritte I und II), der das Enzym nach der Oxidation durch das Wasserstoffperoxid wieder in seine katalytisch wirksame Ausgangsform überführt (Teilschritt II). Aufgrund seiner Stabilität wird in diesem Falle Hydrochinon als Hydriddonor ausgewählt, das durch die Enzymreaktion in p-Benzochinon überführt wird (Teilschritt II). Das so erzeugte p-Benzochinon wird an der Elektrode unter Abgabe zweier Elektronen wieder zu Hydrochinon hydriert (Teilschritt III) und somit durch den entstandenen Strom elektrochemisch detektiert.

Prinzipiell problematisch ist dieses Detektionsverfahren an Edelmetallelektroden, da H₂O₂ als elektrochemisch aktive Substanz einen eigenen Reduktionsstrom an diesen Elektroden erzeugt. Um sicherzustellen, daß die Detektion nicht durch eine Substratlimitierung beeinflußt und damit ein falsches negatives Ergebnis detektiert wird, liegt das H₂O₂ am Anfang der Enzymreaktion, verglichen mit dem neugebildeten p-Benzochinon, als Enzymsubstrat in einem großen Überschuß vor. Entsprechend werden als Elektrodenmaterialien für den erfindungsgemäßen Hybridtransducer spezielle Carbon- und Widerstandspasten ausgewählt. Diese erlauben einen empfindlichen und selektiven Nachweis von p-Benzochinon in der Gegenwart von einem mehr als 1000-fachen Überschuß der genannten Substratkomponenten. Die mit den erfindungsgemäßen Materialien produzierten Elektroden zeigen ähnliche Eigenschaften wie klassische glassy Carbon-Elektroden, die aber im Gegensatz zu Platin- und Goldelektroden nicht im Siebdruck produzierbar sind. Ferner ist ersichtlich, daß aus den genannten Gründen Edelmetallelektroden, wie z.B. Gold-Dickschichtsensoren, nicht optimal als "Peroxidase-Detektor" einsetzbar sind, da das Verhältnis zwischen enzymatischen und nichtenzymatischen Signalen mit steigender Substratkonzentration immer ungünstiger wird und praktisch eine Messung bei optimaler Substratkonzentration, die ca. 1 mM H₂O₂ beträgt, nicht mehr möglich ist.

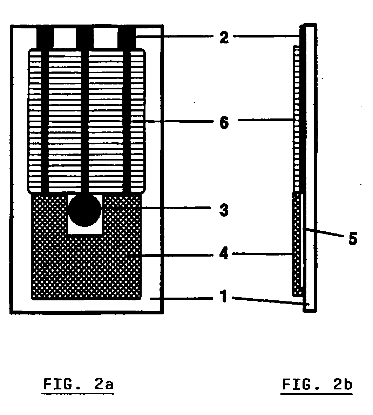

Die Figuren 2a und 2 b zeigen den prinzipiellen Aufbau eines Dickschichtsensors in Draufsicht und in Querschnittsansicht. Auf einem Träger 1, beispielsweise einem Keramikträger, sind Leiterbahnen 2 zum Anlegen der Potentiale und Ableiten des Messignals aufgebracht, die zu einer im allgemeinen großflächigen sensitiven Elektrode 4 und einer Referenzelektrode 3 führen. Die sensitive Elektrode 4 ist aus einer Widerstandspaste entsprechend den bereits angesprochenen Auswahlvorschriften hergestellt. Zur Verbesserung der Leitfähigkeit der sensitiven Elektrode 4 wird zwischen dieser und dem Träger 1 in dieser Ausführungsform eine Goldschicht 5 angeordnet. Andere gut leitende Materialien, beispielsweise Platin etc., sind ebenfalls verwendbar. Ferner sind die Leiterbahnen 2 noch mit einer Isolierung 6 versehen.

### Beispiel 1 : Carbonpaste Ercon C-458

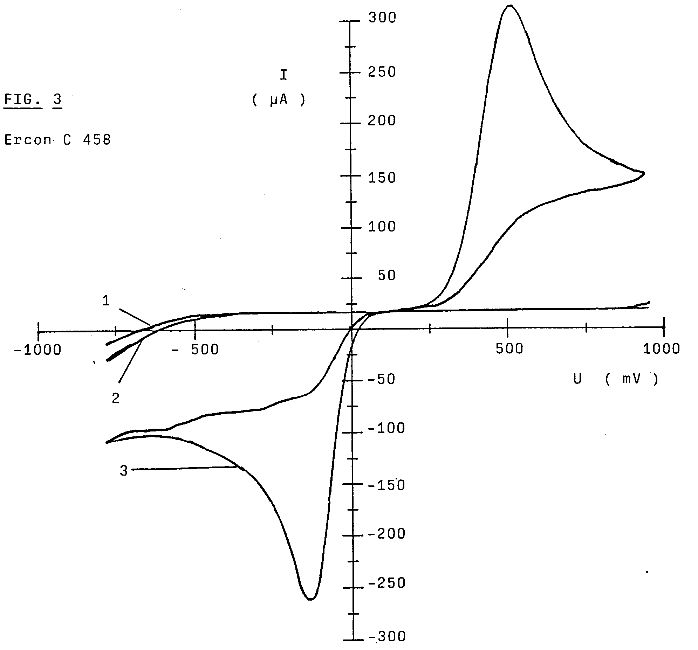

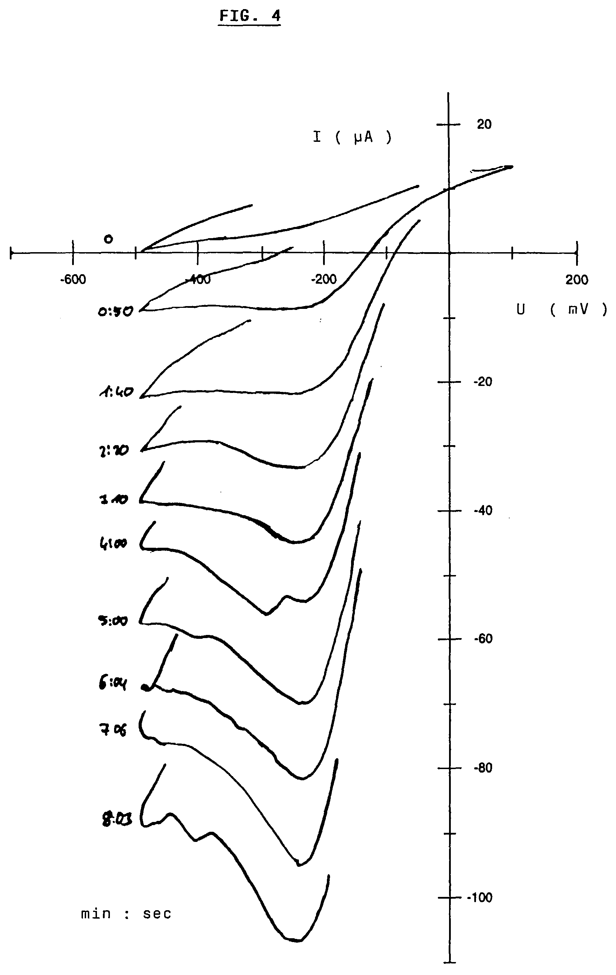

Die Figuren 3 und 4 zeigen Messungen eines Dickschichtsensors mit einer aus der Carbonpaste Ercon C-458 hergestellten selektiven Elektrode. Dabei zeigt Fig. 3 ein cyclisches Voltamogramm, wobei Kurve 1 eine Messung eines 0,1 M KPi-Puffers (KPi = Kaliumphosphat inorganisch) bei einem pH-Wert von 6.0, die Kurve 2 eine Messung nach Zugabe von 1mM H₂O₂ zu dem Puffer, und die Kurve 3 eine Messung nach Zugabe von 1mM p-Benzochinon und 1 mM Hydrochinon zeigt. Dabei ist der Peak bei negativem Potential der interessierende p-Benzochinonpeak, während der Peak im positiven Quadranten dem Hydrochinon zuzuordnen ist. Es ist deutlich zu erkennen, daß bei einem Potential von ca -150mV das p-Benzochinon ein Peak oder Maximum aufweist. gleichzeitig sind die Ströme des Puffers und des Wasserstoffperoxids positiv und klein, d.h. in der Umgebung des Peaks ist ein guter Signal-Rauschabstand gegeben. Mit anderen Worten, die verwendete Widerstandspaste sieht bei dem negativen Potential nur das p-Benzochinon und so gut wie keine Anteile von H₂O₂. Das Hydrochinon wird in diesem Potentialbereich ebenfalls von dem Sensor nicht detektiert.

Fig. 4 zeigt den Signalanstieg eines Dickschichtsensors mit Elektroden der Carbonpaste Ercon C 458 entsprechend der Fig. 3 nach Zugabe gelöster Peroxidase. Die Substratlösung enthält 1 mM H₂O₂, 1 mM Hydrochinon und als Enzym wurde 10 mU/ml Peroxidase zugegeben. Der Meßzyklus hat die Parameter - 650 mV - +1000 mV mit einer Rate von 100 mV pro Sekunde, wobei eine Ag/AgCl Referenzelektrode verwendet wurde. Zu beobachten ist das deutliche Anwachsen des p-Benzochinonpeaks von dem Beginn der Messung (t=0) bis zu dem Maximum nach ca 8 Minuten (t=8 min).

### Beispiel 2: Metalloxidpaste Du Pont QS 872

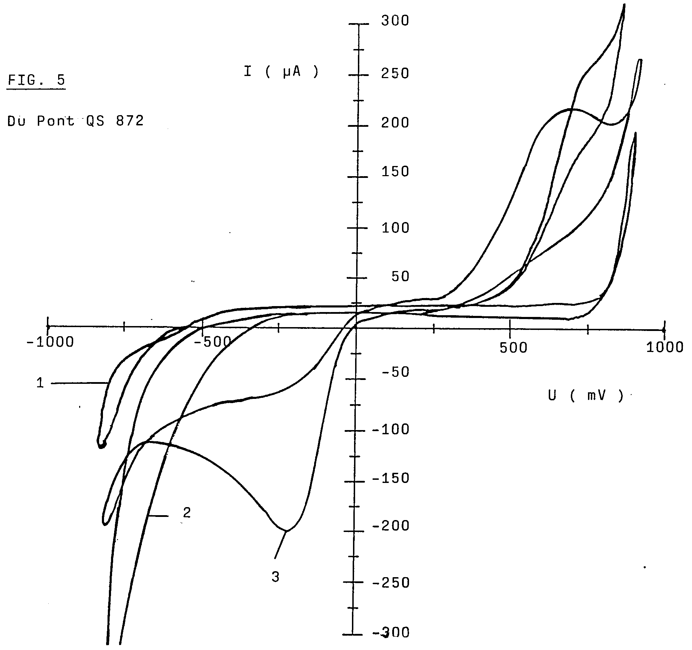

Fig. 5 zeigt ein cyclisches Voltamogramm eines Dickschichtsensors mit einer Elektrode aus der Widerstandspaste Du Pont QS 872, die eine Blei-Rutenium-Dioxid-Paste ist. Die Parameter der Kurven 1, 2, 3 der Fig. 5 entsprechen denjenigen der Fig. 3. Auch hier ist zu sehen, daß im Potentialbereich von ca -250 mV der Sensor nur das p-Benzochinon bei guten Signal-Rauschabstand detektiert.

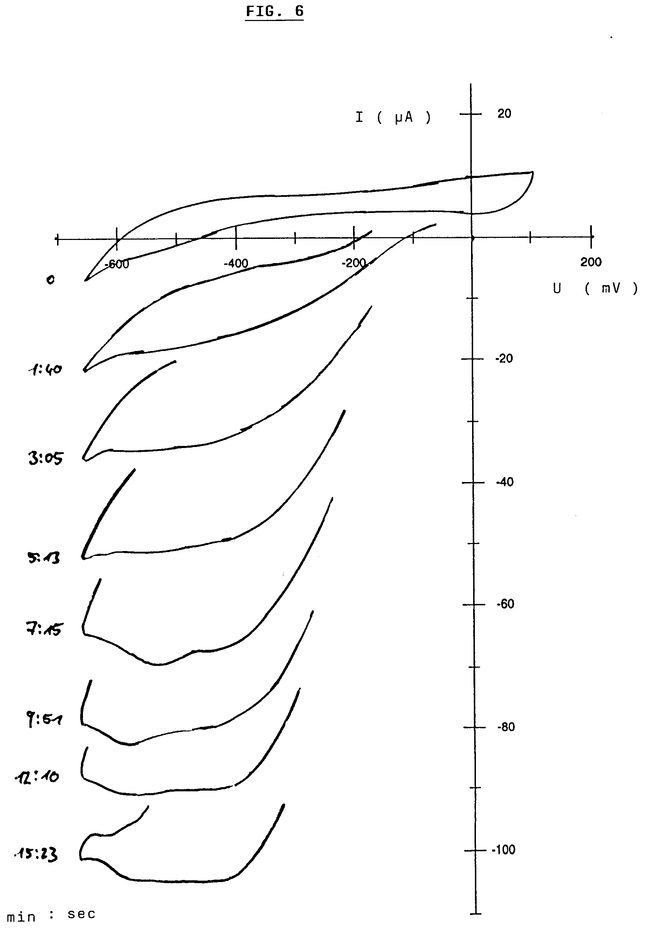

Fig. 6 zeigt den Signalanstieg nach Zugabe gelöster Peroxidase im Falle der Du Pont Widerstandspaste QS 872. Die Parameter der Messung entsprechen denjenigen der Fig. 4. Nach ca 15 Minuten ist der p-Benzochinonpeak voll ausgeprägt.

### Beispiel 3: Carbonpaste Acheson Electrodag 421 SS

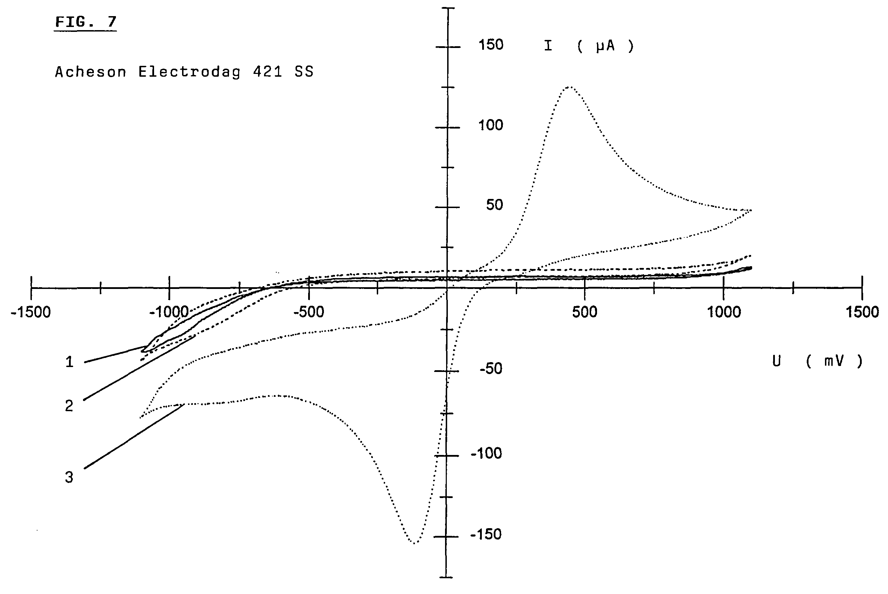

Fig. 7 zeigt ein cyclisches Voltamogramm eines Dickschichtsensors mit einer Elektrode aus der Carbonpaste Acheson Electrodag 421 SS. Die Kurve 1 zeigt den KPi Puffer bei einem pH-Wert von 5.5, die Kurve 2 den Meßverlauf nach Zugabe von 2 mM H₂O₂, und die Kurve 3 den Verlauf nach Zugabe von 1 mM p-Benzochinon. Es ergeben sich bei einem negativen Potential von ca. -150 mV die gleichen Aussagen wie in den Beispielen 1 und 2.

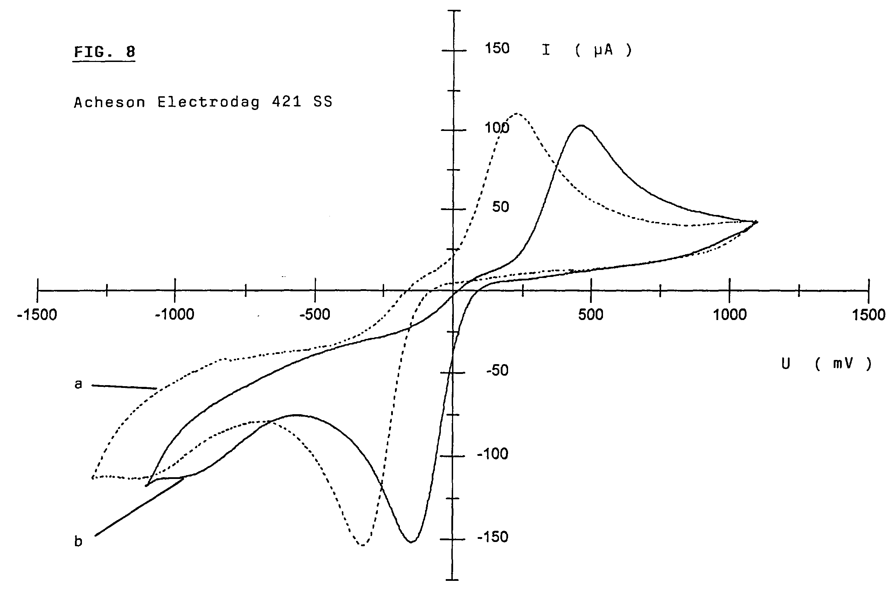

Fig. 8 zeigt die Verschiebung der zyklischen Voltamogramme eines Dickschichtsensors nach Fig. 7 bei der Verwendung unterschiedlicher Referenzelektroden. Dabei zeigt die Kurve a den Verlauf bei der Verwendung einer Platinreferenzelektrode und die Kurve b den Verlauf bei der Verwendung einer Ag/AgCl -Referenzelektrode bei Versuchsbedingungen von 1mM p-Benzochinon und KPi pH-Wert von 5.5. Der Versuch zeigt, daß die Verwendung unterschiedlicher Referenzelektroden nur eine Verschiebung des Kurvenverlaufs bewirkt.

### Beispiel 4: Carbonpaste Ercon G-448

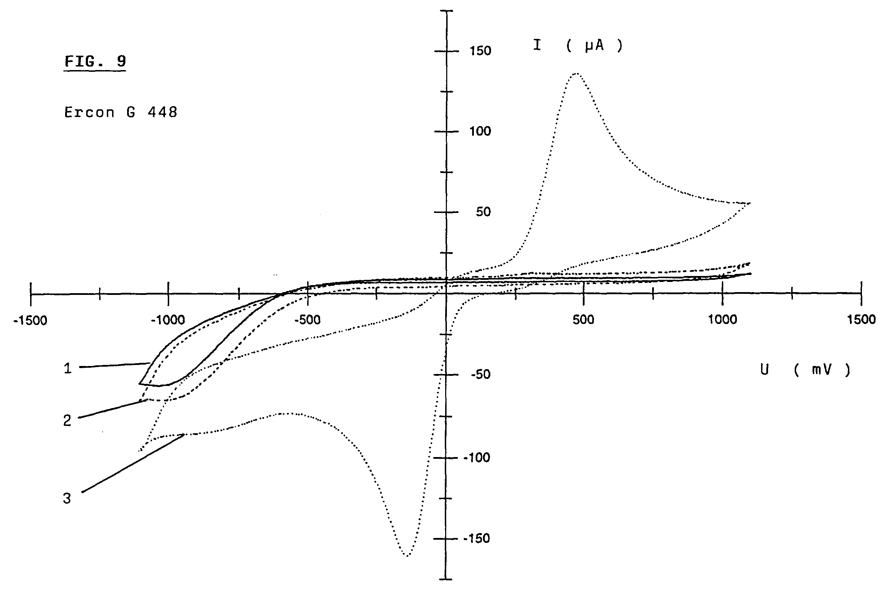

Fig. 9 zeigt ein zyklisches Voltamogramm eines Dickschichtsensors unter Verwendung der Carbonpaste Ercon G-448, wobei unter der selektiven Elektrode zur Erhöhung der Leitfähigkeit eine Goldschicht angeordnet ist. Die Definitionen der Kurvenparameter entsprechen denjenigen des Beispiels 3. Der Versuch ergibt dieselbe Aussage wie die Beispiele 1 -3.

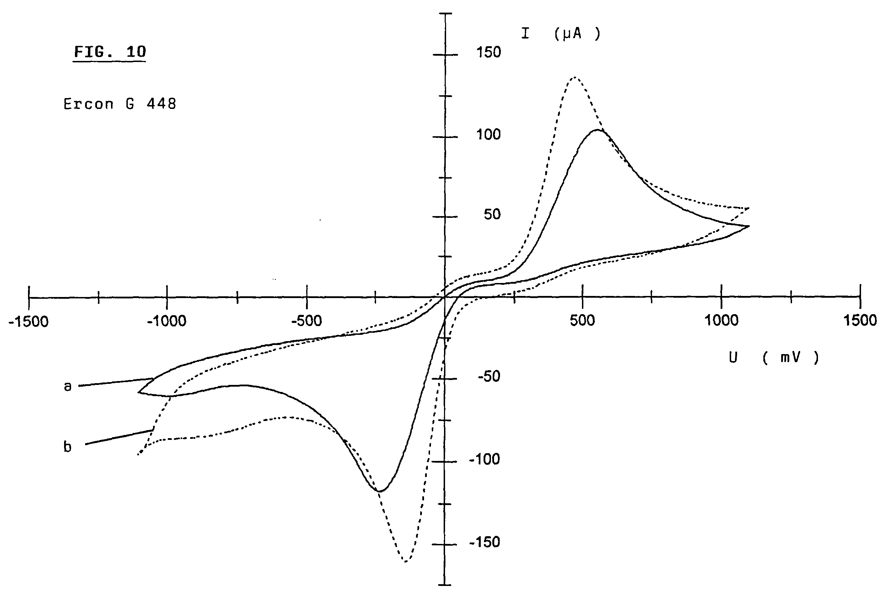

Fig. 10 zeigt eine Gegenüberstellung der zyklischen Voltamogramme eines Dickschichtsensors nach Fig. 9, wobei einmal der Sensor eine Goldschicht aufweist (Kurve b) und einmal ein Sensor ohne Goldschicht (Kurve a) verwendet wird. Es ist deutlich zu erkennen, daß die Verwendung einer Goldschicht eine Erhöhung des Meßsignals und damit eine Erhöhung der Sensitivität des Sensors bewirkt.

### Beispiel 5: Carbonpaste Ercon G-450

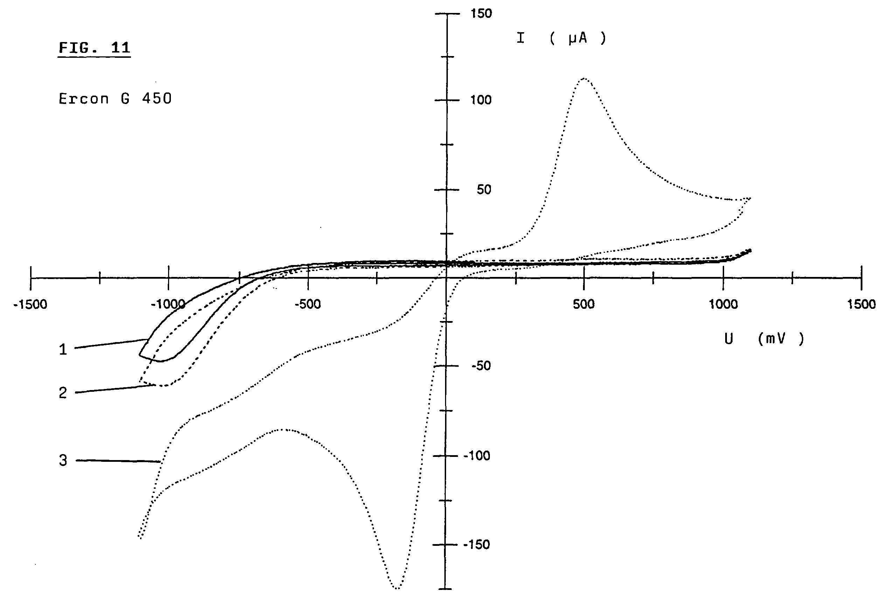

Fig. 11 zeigt ein zyklisches Voltamogramm eines Dickschichtsensors unter Verwendung der Carbonpaste Ercon G-450 mit einer Goldschicht, wobei die Versuchsbedingungen die des Beispiels 4 sind. Die Ergebnisse der Beispiele 1 - 4 treffen auch hier zu.

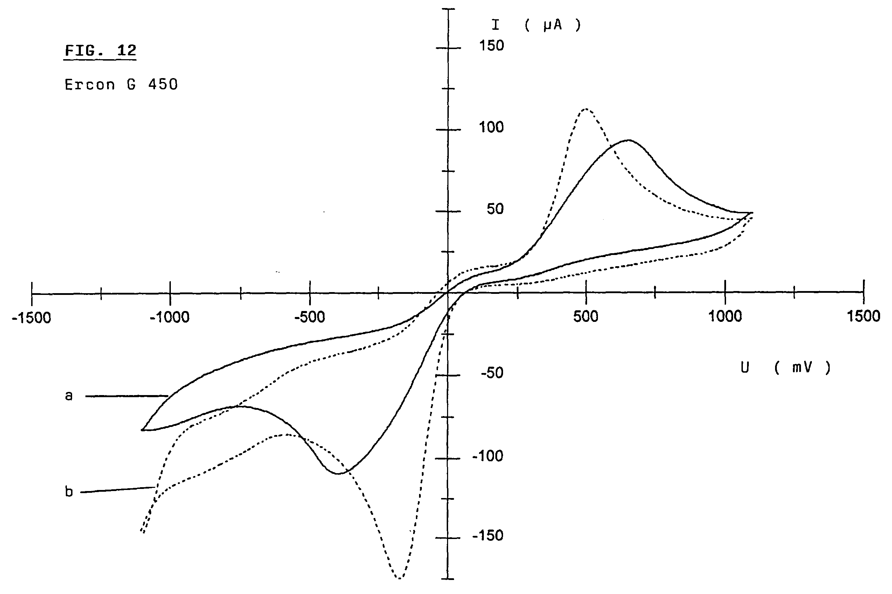

Fig. 12 zeigt eine Gegenüberstellung der zyklischen Voltamogramme des Dickschichtsensors nach Fig. 11 einmal mit (Kurve b) und einmal ohne Goldschicht (Kurve a). Auch hier wird deutlich, daß der Sensor mit der Goldschicht (Kurve b) ein besseres Meßsignal liefert.

### Beispiel 6: Carbonpaste Ercon Exp #551112

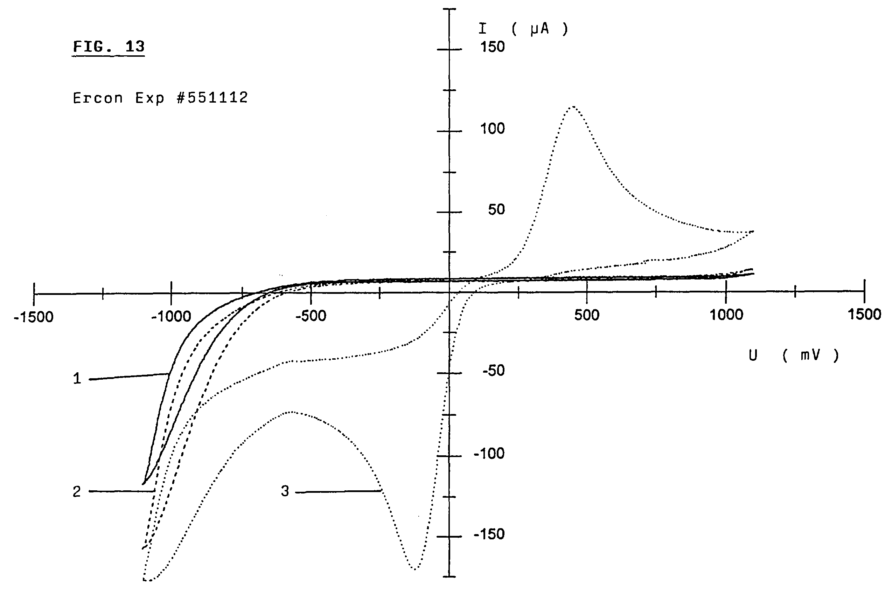

Fig. 13 zeigt ein zyklisches Voltamogramm eines Dickschichtsensors unter Verwendung der Carbonpaste Ercon Exp #551112 mit einer Goldschicht und den Versuchsbedingungen der Beispiele 4 und 5. Hier ist der Sensor ebenfalls bei einem Potential von ca -150 mV nur für p-Benzochinon empfindlich.

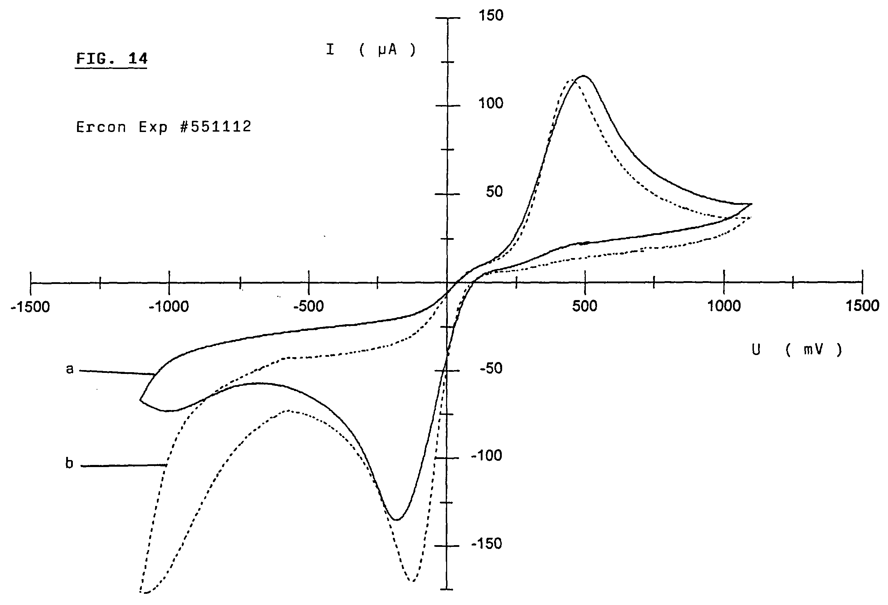

Fig. 14 schließlich zeigt eine Gegenüberstellung der zyklischen Voltamogramme des Dickschichtsensors nach Fig. 13 einmal mit (Kurve b) und einmal ohne (Kurve a) Goldschicht. Auch hier wird deutlich, daß der Sensor mit der Goldschicht (Kurve b) ein besseres Meßsignal liefert und daher eine höhere Sensitivität aufweist.

### Bezugszeichenliste

- 1: Träger
- 2: Leiterbahn
- 3: Referenzelektrode
- 4: selektive Elektrode
- 5: leitende Schicht
- 6: Isolierung

## Patentansprüche

1. Dickschichtsensor zur Bestimmung der Peroxidaseaktivität mit einer sensitiven Elektrode (4), **dadurch gekennzeichnet**, daß die Elektrode (4) aus einer Widerstandspaste hergestellt wird, wobei die Widerstandspaste dadurch bestimmt wird, daß sie geringe Menge p-Benzochinon in der Gegenwart hoher Konzentrationen von H₂O₂ messen kann.

2. Dickschichtsensor nach Anspruch 1, **dadurch gekennzeichnet**, daß die Widerstandspaste ferner dadurch bestimmt wird, daß die Widerstandspaste geringe Mengen p-Benzochinon in der Gegenwart hoher Konzentrationen von Hydrochinon messen kann.

3. Dickschichtelektrode nach Anspruch 1 oder 2, **dadurch gekennzeichnet**, daß das Elektrodenpotential zur Messung des p-Benzochinons bei der Bestimmung der Widerstandspaste zwischen - 10 mV und -700mV liegt.

4. Dickschichtelektrode nach Anspruch 3, **dadurch gekennzeichnet**, daß das Elektrodenpotential ca -150mV beträgt.

5. Dickschichtsensor nach einem der Ansprüche 1 - 4, **dadurch gekennzeichnet**, daß die Widerstandspaste eine graphithaltige Paste ist.

6. Dickschichtsensor nach Anspruch 5, **dadurch gekennzeichnet**, daß die Graphitpaste einen relativ niedrigen eigenen Widerstand aufweist.

7. Dickschichtsensor nach einem der Ansprüche 1 - 4, **dadurch gekennzeichnet**, daß die Widerstandspaste eine Metalloxidpaste ist, die nicht H₂O₂-empfindlich ist.

8. Dickschichtsensor nach einem der vorangegangenen Ansprüche, **dadurch gekennzeichnet**, daß die Metalloxidpaste eine Blei-Rutenium-Dioxidpaste ist.

9. Dickschichtsensor nach einem der vorangegangenen Ansprüche, **dadurch gekennzeichnet**, daß die Widerstandspasten siebdruckfahig sind.

10. Dickschichtelektrode nach Anspruch 9, **dadurch gekennzeichnet**, daß die Elektrode (4) auf einem Träger (1) angeordnet ist.

11. Dickschichtsensor nach Anspruch 10, **dadurch gekennzeichnet**, daß die Elektrode (4) auf einer gut leitfähigen Schicht (5) aufgebracht ist, die zwischen der Elektrode (4) und dem Träger (1) angeordnet ist.

12. Dickschichtsensor nach Anspruch 11, **dadurch gekennzeichnet**, daß die gut leitfähige Schicht (5) eine Goldschicht ist.

13. Verfahren zur Herstellung eines Dickschichtsensors zur Bestimmung der Peroxidaseaktivität nach einem der Ansprüche 1 - 10, wobei die Widerstandspaste mittels Siebdrucktechnik auf einen Träger (1) aufgebracht wird, und die Elektrode (4) durch anschließendes Trocknen oder Brennen der Widerstandspaste gebildet wird.

14. Verfahren nach Anspruch 13, **dadurch gekennzeichnet**, daß der Träger (1) mit einer gut leitfähigen Schicht (5), vorzugsweise einer Goldschicht dort versehen wird, wo die Widerstandspaste zur Bildung der sensitiven Elektrode (4) aufgebracht wird.

15. Verfahren nach Anspruch 13 oder 14, **dadurch gekennzeichnet**, daß bei Graphitpasten die Trocknungstemperatur mindestens 80° C beträgt.

16. Verfahren nach Anspruch 15, **dadurch gekennzeichnet**, daß die Trocknungstemperatur (Einbrenntemperatur) vorzugsweise ca. 150° C beträgt.

17. Verfahren nach Anspruch 13 oder 12, **dadurch gekennzeichnet**, daß bei Metalloxidpasten die Brenntemperatur 700°C - 900°C, vorzugsweise ca. 800°C, beträgt.
